(19) Europäisches Patentamt
European Patent Office
Office européen des brevets

(11) Veröffentlichungsnummer: **0 186 834**
**B1**

(12) # EUROPÄISCHE PATENTSCHRIFT

(45) Veröffentlichungstag der Patentschrift: **30.08.89**

(51) Int. Cl.⁴: **C 07 J 41/00**, A 61 K 31/575

(21) Anmeldenummer: **85115925.1**

(22) Anmeldetag: **13.12.85**

(54) **13alpha-Alkyl-17beta-(3-acyloxy-propyl)-gonane.**

**Ein Antrag gemäss Regel 88 auf Berichtigung der Beschreibung liegt vor. Über diesen Antrag wird im Laufe des Verfahrens von der Prüfungsabteilung eine Entscheidung getroffen werden.**

(30) Priorität: **18.12.84 DE 3446661**

(43) Veröffentlichungstag der Anmeldung: **09.07.86 Patentblatt 86/28**

(45) Bekanntmachung des Hinweises auf die Patenterteilung: **30.08.89 Patentblatt 89/35**

(84) Benannte Vertragsstaaten: **AT BE CH DE FR GB IT LI LU NL SE**

(56) Entgegenhaltungen:
**EP-A- 0 057 115**
**EP-A- 0 116 974**
**EP-A- 0 129 499**
**STEROIDS, Band 44, Nr. 4, Oktober 1984, Seiten 349-372, Holden Day, San Francisco, US; GÜNTER NEEF et al.: "New steroids with antiprogestational and antiglucocorticoid activities"**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Neef, Günter, Dr.**
**Darmstädter Strasse 9**
**D-1000 Berlin 15 (DE)**
Erfinder: **Wiechert, Rudolf, Prof.**
**Petzower Strasse 8 A**
**D-1000 Berlin 39 (DE)**
Erfinder: **Beier, Sybille, Dr.**
**Uhlandstrasse 121**
**D-1000 Berlin 31 (DE)**
Erfinder: **Elger, Walter, Dr.**
**Schorlemmer Allee 12 B**
**D-1000 Berlin 33 (DE)**
Erfinder: **Henderson, David, Dr.**
**Jahn Strasse 17**
**D-1000 Berlin 28 (DE)**

**EP 0 186 834 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 13α-Alkyl-17β-(3-acyloxypropyl)-gonane der allgemeinen Formel I,

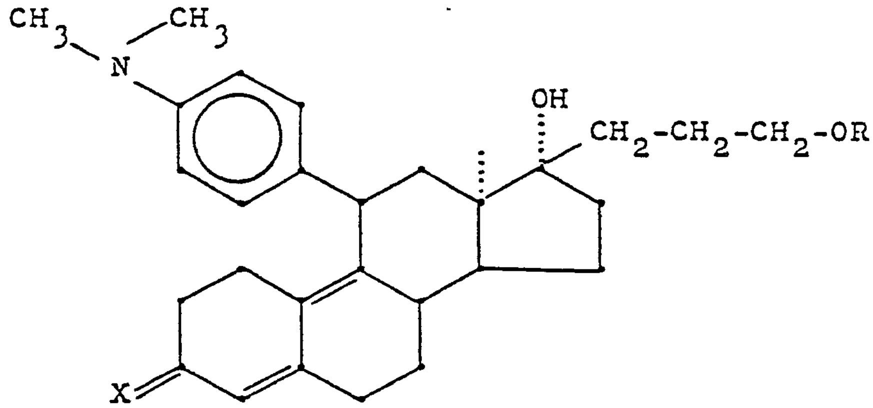

(I)

worin,

R für einen Acylrest mit bis zu 10 C-Atomen und

X für ein Sauerstoffatom oder die Gruppierung N ~ OH stehen, ein Verfahren zu deren Herstellung sowie diese Verbindungen enthaltende pharmazeutische Präparate.

13α-Alkyl-17β-(3-hydroxypropyl)-4,9-gonadien-3-one sind aus der europäischen Patentanmeldung EP-A-0 129 499 (ein Dokument gemäß Art 54(3) EPÜ) bekannt. Diese Verbindungen besitzen eine starke Affinität zum Gestagenrezeptor, ohne selbst gestagene Aktivität zu besitzen. Sie sind kompetitive Antagonisten des Progesterons (Anti-Gestagene) und sind zur Auslösung von Aborten geeignet, da sie das zur Aufrechterhaltung der Schwangerschaft erforderliche Progesteron vom Rezeptor verdrängen. Die Verbindungen sind deshalb wertvoll und interessant im Hinblick auf ihre Verwendung zur postcoitalen (p.c.) Fertilitätskontrolle.

Es wurde nun gefunden, daß durch Überführung dieser Verbindungen in die 13α-Alkyl-17β-(3-acyloxypropyl)-gonane der allgemeinen Formel I überraschenderweise ihre Bioverfügbarkeit wesentlich verbessert werden kann. Sie weisen daher bei gleicher oder verstärkter Wirkung eine wesentlich verlängerte Wirkungsdauer im Vergleich zu den vorbekannten 13α-Alkyl-17β-(3-hydroxypropyl)-4,9-gonadien-3-onen auf. Als weiteren Vorteil stellte sich heraus, daß die neuen Verbindungen ausgezeichnete Kristallisationseigenschaften besitzen. Die Herstellung reiner Wirksubstanzen bereitet daher bei ihnen sehr viel weniger Schwierigkeiten als im Falle der 13α-Alkyl-17β-(3-hydroxypropyl)-4,9-gonadien-3-one.

Weiterhin besitzen sie eine ausgezeichnete chemische Stabilität. Man kann sie problemlos bei Raumtemperatur über einen größeren Zeitraum ohne Zersetzung lagern.

Der Acylrest R der allgemeinen Formel I soll bis zu 10 C-Atomen enthalten. Als Acylreste kommen beispielsweise infrage der Formyl-, Acetyl-, Propionyl-, Butyryl-, Isobutyryl-, Valeryl-, Isovaleryl-, Glycoloyl-, Cyclopentylacetyl-, Mono-, Di-, Trichloracetyl-, Benzoyl-, Trifluormethylbenzoyl- und Nicotinoylrest; bevorzugt sind der Acetyl- und der Benzoylrest.

X steht für ein Sauerstoffatom oder die Gruppierung N ~ OH, wobei der Hydroxyimino-Rest syn- oder antiständig sein kann.

Die Herstellung der erfindungsgemäßen Verbindungen der allgemeinen Formel I erfolgt, indem man eine Verbindung der Formel II

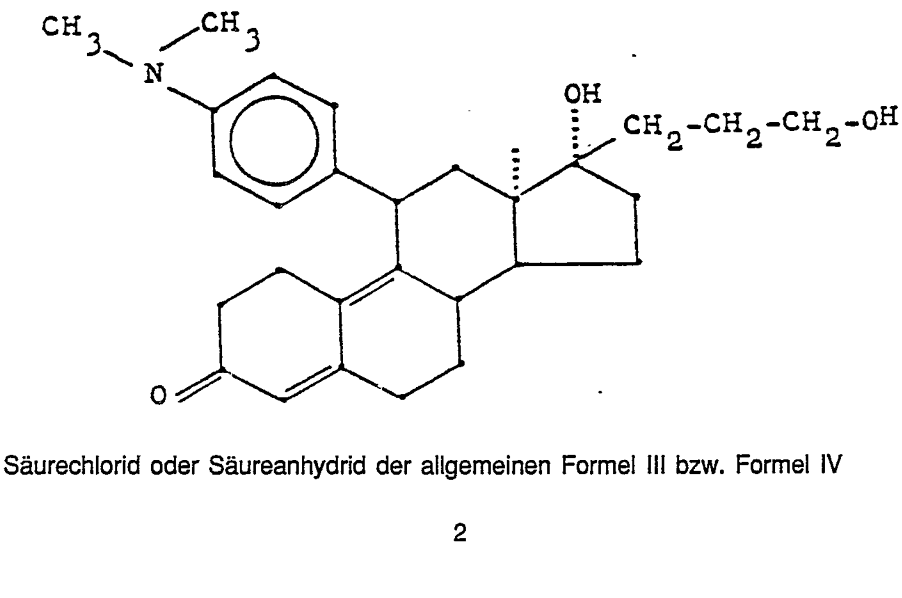

(II)

mit einem Säurechlorid oder Säureanhydrid der allgemeinen Formel III bzw. Formel IV

$$\begin{array}{c} O \\ \| \\ R-C-Cl \end{array} \qquad (III)$$

$$\begin{array}{c} O \\ \| \\ (R-C)_2O \end{array} \qquad (IV)$$

worin R die obige Bedeutung hat, in Gegenwart von Basen bei Temperaturen zwischen 0 und 60 °C umsetzt und gegebenenfalls anschließend mit Hydroxylamin-hydrochlorid in Gegenwart von tertiären Aminen bei Temperaturen zwischen —20 und +40 °C umsetzt.

Die bevorzugten Basen bei der Veresterung sind tertiäre Amine wie beispielsweise Pyridin. Das bevorzugte tertiäre Amin bei der Umsetzung zur Herstellung der Oxime ist Pyridin.

Weitere geeignete tertiäre Basen sind beispielsweise Trimethylamin, Triäthylamin, N,N-Dimethylaminopyridin, 1,5-Diazabicyclo [4.3.0]nonen-5(DBN) und 1,5-Diazabicyclo-[5.4.0]undecen-5(DBU).

Die 13α-Alkylgonane der allgemeinen Formel I können in Form pharmazeutischer Präparate Verwendung finden. Die Herstellung der Präparate erfolgt nach an sich bekannten Methoden der Galenik durch Mischen mit organischem oder anorganischem inerten Trägermaterial, welches für die enterale, perkutane oder parenterale Applikation geeignet ist.

Die Dosierung der erfindungsgemäßen Wirkstoffe liegt beim Menschen bei etwa 10 bis 1 000 mg pro Tag. Typische Dosiseinheiten enthalten 10-200 mg Wirkstoff. Die Erfindung soll an Hand der nachfolgenden Beispiele erläutert werden, ohne sie allerdings darauf zu begrenzen.

## Beispiel 1

Eine Lösung von 2,4 g 11β-(4-Dimethylaminophenyl)-17α-hydroxy-13α-methyl-17β-(3-hydroxypropyl)-4,9-gonadien-3-on in 7 ml Pyridin und 14 ml Acetanhydrid wird 14 Stunden bei 25 °C gerührt. Anschließend gießt man in ca. 50 °C warmes Wasser (100 ml), rührt 15 Minuten und extrahiert die erkaltete Emulsion mit Methylenchlorid. Die Methylenchlorid-Phase wird mit $NaHCO_3$-Lösung neutral gewaschen, über $Na_2SO_4$ getrocknet und eingeengt. Kristallisation des Rohprodukts aus Ethylacetat/Diisopropylether ergibt 2,24 g 17β-(3-Acetoxypropyl)-11β-(4-dimethylaminophenyl)-17α-hydroxy-13α-methyl-4,9-gonadien-3-on vom Schmelzpunkt 162-164 °C.

$[\alpha]^{25}_D$+436,5° ($CHCl_3$, c = 0,515).

## Beispiel 2

Zu einer Lösung von 700 mg 11β-(4-Dimethylaminophenyl)-17α-hydroxy-13α-methyl-17β-(3-hydroxypropyl)-4,9-gonadien-3-on in 5 ml Pyridin tropft man unter Eiswasserkühlung 0,36 ml Benzoylchlorid in 4 ml Methylenchlorid. Man rührt 60 Minuten bei +5 °C, gießt dann in gesättigte $NaHCO_3$-Lösung und extrahiert mit Ethylacetat. Kristallisation des Rohprodukts aus Hexan/Diisopropylether ergibt 630 mg 17β-(3-Benzoyloxypropyl)-11β-(4-dimethylaminophenyl)-17α-hydroxy-13α-methyl-4,9-gonadien-3-on vom Schmelzpunkt 114-116 °C.

## Beispiel 3

Eine Lösung von 630 mg 17β-(3-Acetoxypropyl)-11β-(4-dimethylaminophenyl)-17α-hydroxy-13α-methyl-4,9-gonadien-3-on in 10 ml Pyridin wird nach Zusatz von 600 mg Hydroxylamin-hydrochlorid 1,5 Stunden bei 0 °C gerührt. Anschließend gießt man in Eiswasser/gesättigte $NH_4C1$-Lösung und extrahiert mit Ethylacetat. Nach Chromatographie an Kieselgel mit Hexan/Ethylacetat erhält man 410 mg 17β-(3-Acetoxypropyl)-11β-(4-dimethylaminophenyl)-17α-hydroxy-13α-methyl-4,9-gonadien-3-on-anti-oxim vom Schmelzpunkt 201-204 °C.

UV (MeOH) : $\lambda_{max}$ 288 nm (ε = 27 400).

## Beispiel 4

Zusammensetzung einer Tablette mit 17β-(3-Acetoxypropyl)-11β-[(4-N,N-dimethylamino)-phenyl]-17α-hydroxy-13α-methyl-4,9-gonadien-3-on zur oralen Applikation

10 mg 17β-(3-Acetoxypropyl)-11β-[(4-N,N-Dimethylamino)-phenyl]-17α-hydroxy-13α-methyl-4,9-gonadien-3-on
140,5 mg Laktose
69,5 mg Maisstärke
2,5 mg Polyvinylpyrrolidon 25
2,0 mg Aerosil
0,5 mg Magnesiumstearat
———
225.0 mg

## Patentansprüche

1. 13α-Alkylgonane der allgemeinen Formel I

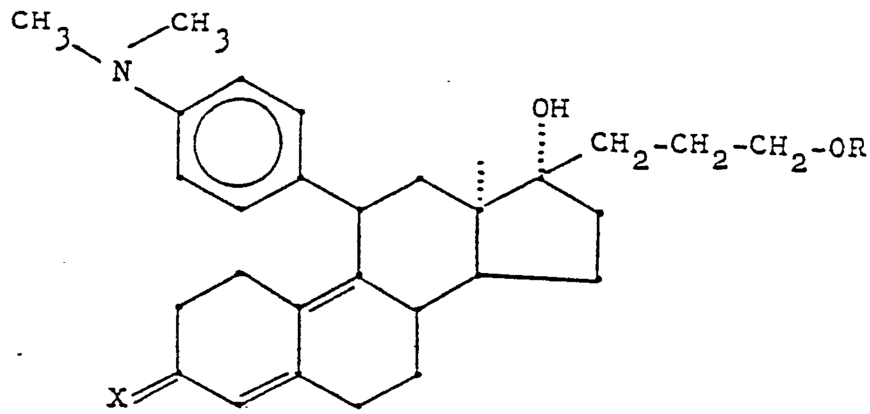

(I)

worin

R für einen Acylrest mit bis zu 10 C-Atomen und

X für ein Sauerstoffatom oder die Gruppierung N ~ OH stehen.

2. 17β-(3-Acetoxypropyl)-11β-(4-dimethylaminophenyl)-17α-hydroxy-13α-methyl-4,9-gonadien-3-on.

3. 17β-(3-Benzoyloxypropyl)-11β-(4-dimethylaminophenyl)-17α-hydroxy-13α-methyl-4,9-gonadien-3-on.

4. 17β-(3-Acetoxypropyl)-11β-(4-dimethylaminophenyl)-17α-hydroxy-13α-methyl-4,9-gonadien-3-on-anti-oxim.

5. Verfahren zur Herstellung von 13α-Alkylgonanen der allgemeinen Formel I

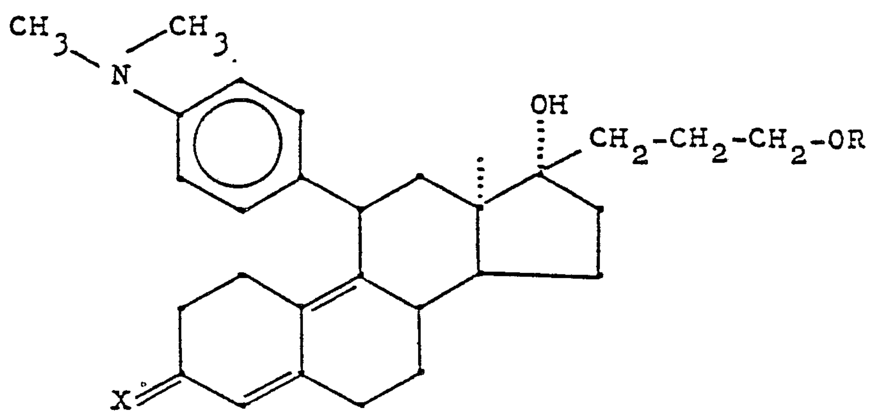

(I)

worin R und X die in Anspruch 1 angegebene Bedeutung haben, dadurch gekennzeichnet, daß man eine Verbindung der Formel II

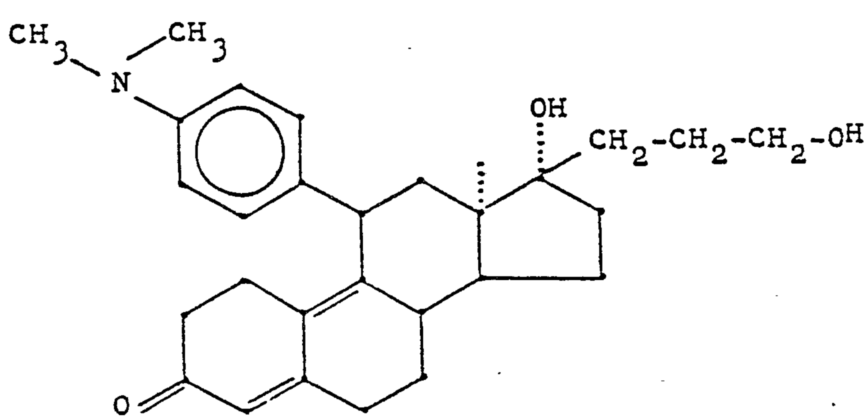

(II)

mit einem Säurechlorid oder Säureanhydrid der allgemeinen Formel III bzw. Formel IV

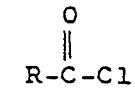

(III)

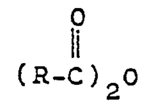

(IV)

worin R die in Anspruch 1 angegebene Bedeutung hat, in Gegenwart von Basen bei Temperaturen zwischen 0 und 60 °C umsetzt und gegebenenfalls anschließend mit Hydroxylamin-hydrochlorid in Gegenwart von tertiären Aminen bei Temperaturen zwischen —20 und +40 °C umsetzt.

6. Pharmazeutische Präparate gekennzeichnet durch den Gehalt an Verbindungen gemäß den Ansprüchen 1 bis 4.

7. Verwendung von Verbindungen gemäß den Ansprüchen 1 bis 4 zur Herstellung von Arzneimitteln.

**Revendications**

1. 13α-Alkylgonanes répondant à la formule générale

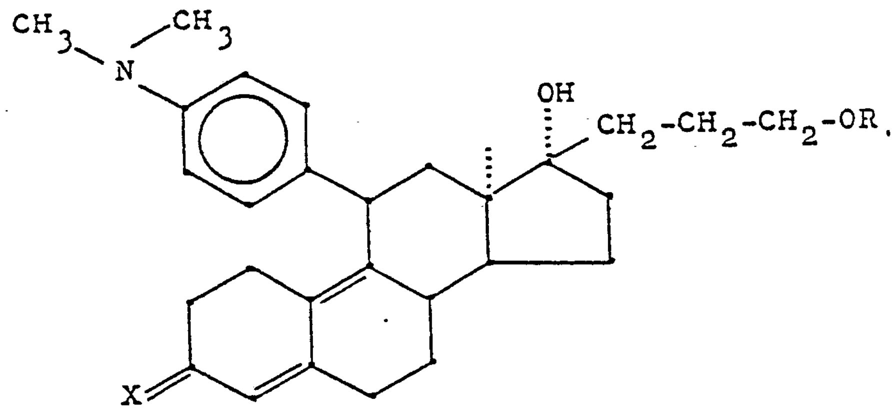

(I)

dans laquelle

R représente un radical acyle comportant jusqu'à 10 atomes de carbone, et

X représente un atome d'oxygène ou le groupement N ~ OH.

2. La 17β-(3-acétoxypropyl)-11β-(4-diméthylaminophényl)-17α-hydroxy-13α-méthyl-4,9-gonadién-3-one.

3. La 17β-(3-benzoyloxypropyl)-11β-(4-diméthylaminophényl)-17α-hydroxy-13α-méthyl-4,9-donadién-3-one.

4. La 17β-(3-acétoxypropyl)-11β-(4-diméthylaminophényl)-17α-hydroxy-13α-méthyl-4,9-gonadién-3-one-anti-oxime.

5. Procédé de préparation de 13α-alkylgonanes répondant à la formule générale I

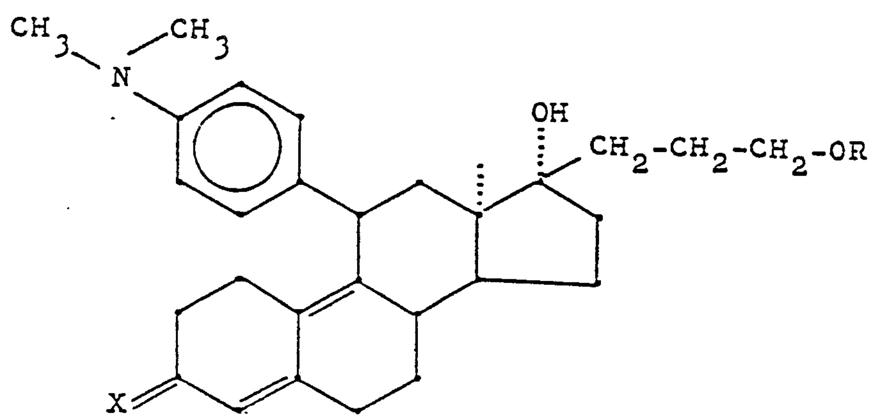

(I)

dans laquelle R et X ont la même signification que celle donnée dans la revendication 1, caractérisé en ce que, à des températures comprises entre 0 et 60 °C, on fait réagir un composé de la formule II

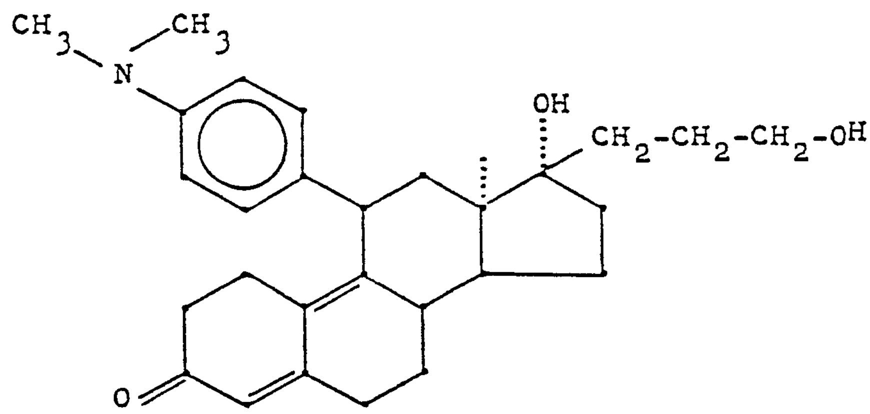

(II)

avec un chlorure d'acide ou un anhydride d'acide répondant aux formules générales III ou IV

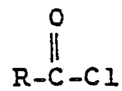

(III)

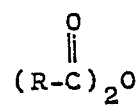

(IV)

où R à la même signification que celle donnée dans la revendication 1, en présence de bases, et en ce qu'on fait éventuellement réagir ensuite avec du chlorhydrate d'hydroxylamine en présence d'amines tertiaires, à des températures comprises entre —20 et +40 °C.

6. Compositions pharmaceutiques caractérisées par une teneur en composés suivant l'une des revendications 1 à 4.

7. Utilisation de composés suivant l'une des revendications 1 à 4, pour la préparation de médicaments.

## Claims

1. 13α-alkylgonanes of general formula I

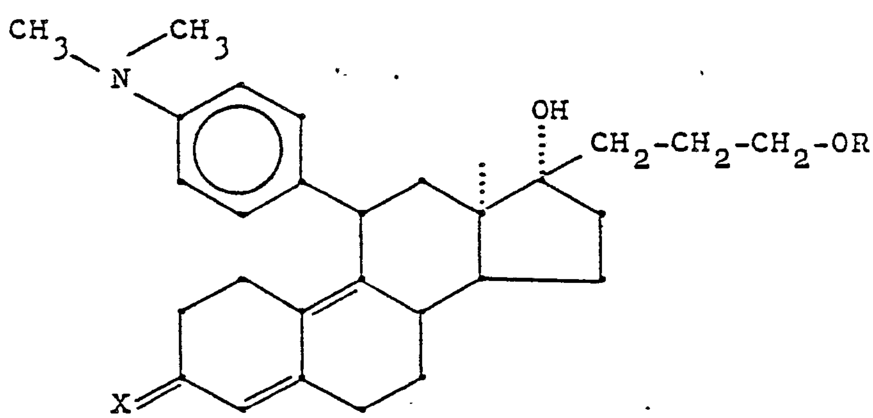

(I)

wherein

R represents an acyl radical having up to 10 C atoms and

X represents an oxygen atom or the grouping N ~ OH.

2. 17β-(3-acetoxypropyl)-11β-(4-dimethylaminophenyl)-17α-hydroxy-13α-methyl-4,9-gonadien-3-one.

3. 17β-(3-benzoyloxypropyl)-11β-(4-dimethylaminophenyl)-17α-hydroxy-13α-methyl-4,9-gonadien-3-one.

4. 17β-(3-acetoxypropyl)-11β-(4-dimethylaminophenyl)-17α-hydroxy-13α-methyl-4,9-gonadien-3-one anti-oxime.

5. Process for the preparation of 13α-alkylgonanes of general formula I

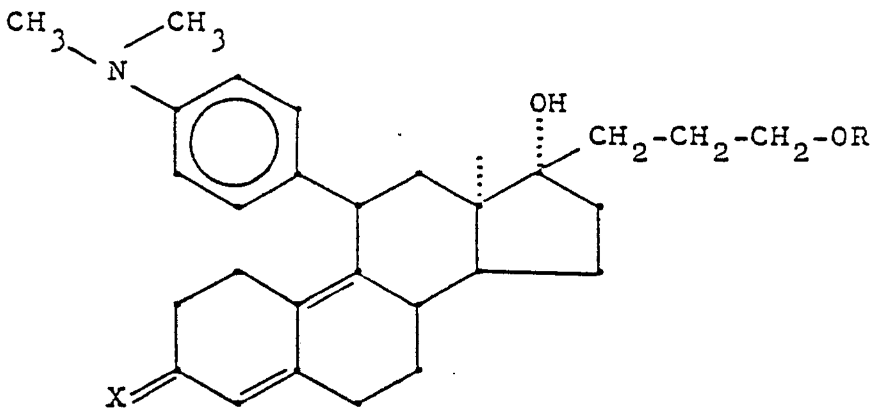

(I)

wherein R and X have the meanings indicated in claim 1, characterised in that a compound of formula II

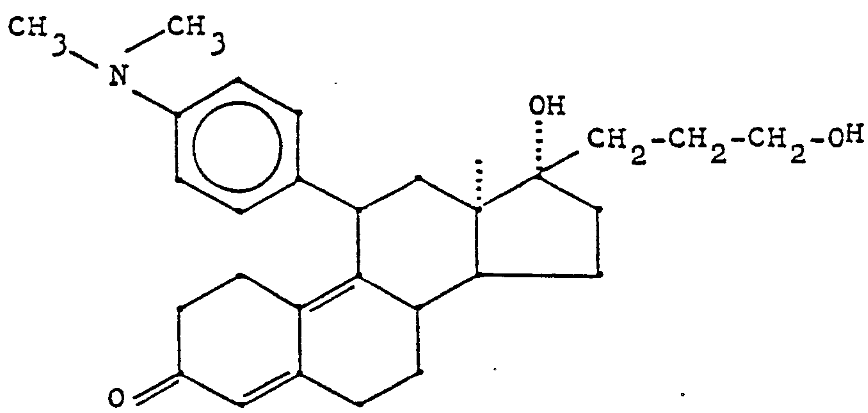

(II)

is reacted with an acid chloride or acid anhydride of general formula III or IV respectively

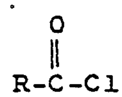

(III)

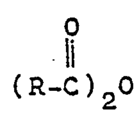

(IV)

wherein R has the meaning indicated in claim 1, in the presence of bases at temperatures between 0 and 60 °C and, if desired, the product is subsequently reacted with hydroxylamine hydrochloride in the presence of tertiary amines at temperatures between —20 and +40 °C.

6. Pharmaceutical preparations, characterised in that they contain compounds according to claims 1 to 4.

7. Use of compounds according to claims 1 to 4 for the manufacture of medicaments.